Europäisches Patentamt

European Patent Office

Office européen des brevets

(11) Publication number: **0 526 974 B1**

# EUROPEAN PATENT SPECIFICATION

(45) Date of publication of patent specification: **15.11.95**  (51) Int. Cl.6: **C07C 51/12**

(21) Application number: **92305624.6**

(22) Date of filing: **19.06.92**

(54) **Process for the preparation of acetic acid.**

(30) Priority: **21.06.91 JP 175818/91**
**12.06.92 JP 177809/92**

(43) Date of publication of application:
**10.02.93 Bulletin 93/06**

(45) Publication of the grant of the patent:
**15.11.95 Bulletin 95/46**

(84) Designated Contracting States:
**DE FR GB**

(56) References cited:
**GB-A- 1 277 242**
**GB-A- 1 501 892**

**PROCEEDINGS OF 9TH INTERNATIONAL CA-
TALYSIS CONGRESS, vol. 3, 1988, Tokyo, JP,
pages 1051 - 1058, K. FUJIMOTO et al, "Effect
of hydrogen on vapor phase carbonylation
of methanol"**

(73) Proprietor: **TOKYO GAS CO., LTD.**
**5-20, Kaigan 1-chome**
**Minato-ku**
**Tokyo 105 (JP)**

(72) Inventor: **Yamaseki, Kenichi**
**1-6-3, Mure**
**Mitaka-shi,**
**Tokyo (JP)**
Inventor: **Konishi, Yasuo**
**42-2, Miyagaya,**
**Nishi-ku**
**Yokohama-shi,**
**Kanagawa-ken (JP)**
Inventor: **Uchida, Hiroshi**
**3-2-15-106, Azamino,**
**Midori-ku**
**Yokohama-shi,**
**Kanagawa-ken (JP)**

(74) Representative: **Harrison, David Christopher
et al**
**MEWBURN ELLIS**
**York House**
**23 Kingsway**
**London WC2B 6HP (GB)**

Rank Xerox (UK) Business Services
(3.10/3.09/3.3.3)

**Description**

This invention relates to a process for preparing acetic acid from methanol and carbon monoxide or a mixed gas of carbon monoxide with hydrogen.

The so-called synthesis gas obtained by partial combustion reactions, steam reforming, etc. of coal, petroleum, natural gas, etc. and which is a gaseous mixture of high content of hydrogen and carbon monoxide is known in the art to be used as a starting material for the preparation of acetic acid. It usually contains hydrogen in an amount of 20 to 75% by volume.

For example, Japanese Patent Publication No. 43767/85 discloses an alcohol carbonylation catalyst formed by supporting nickel, cobalt, compounds thereof and the like on a carbon support, and further discloses a process for preparing acetic acid which comprises subject methanol and carbon monoxide to a vapor phase catalytic reaction in the presence of the above catalyst and an iodine compound promoter, but neither teaches nor suggests the use of a mixed gas of high content hydrogen with carbon monoxide, i.e. the so-called synthesis gas as a starting material. Nor does it teach or suggest the use of a carbon-supported rhodium catalyst. As a result there is an unsatisfactory selectivity and yield of acetic acid.

In Chemistry Letters, pp. 895-898, 1987, Fujimoto et al. reports the promotion effect of hydrogen on vapor phase carbonylation of methanol over a nickel on active carbon catalyst, and further reports that the yield of acetic acid reaches a maximum level at an $H_2/CO$ ratio of 0.1 or above. He shows that presence of hydrogen in amounts such as a $H_2/CO$ ratio of 0.1 or above remarkably improves the yield of acetic acid compared with the use of a hydrogen-free carbon monoxide, but with unsatisfactory results.

In the Proceedings of 9th International Catalysis Congress, 3, pages 1051-1058 (1988), Fujimoto et al. teach that activity of methanol carbonylation on Rh is the highest in comparison with other metals such as Ni, Pd, Co and the like, and that addition of hydrogen as in the reaction conditions of 523°K, 11 atm, W//F = 5 g.h/mol (Rh: 1 g.h/mol), and $CO/MeOH/MeI/H_2$ = 50/9/1/(19) molar ratio improves activity of carbonylation compared with the case where no hydrogen is added, but shows unsatisfactory yields of acetic acid.

Japanese Patent Publication No 3334/72 discloses a process for preparing acetic acid which comprises effecting methanol carbonylation in the liquid phase in the presence of a rhodium complex catalyst and a promoter comprising methyl iodide or hydrogen iodide to obtain acetic acid at high yields, but has the disadvantages that the reaction system is so corrosive that use of costly corrosion-resistant material is required, that by-production of methane is increased, and so forth, and neither teaches nor suggests the use of a mixed gas of high content hydrogen with carbon monoxide, i.e. the so-called synthesis gas as a starting material.

Japanese Patent Application Laid-Open No. 299248/89 discloses a process for the production from methanol of acetic acid which process comprises contacting methanol with a gaseous mixture of carbon monoxide and hydrogen in the presence of a catalyst comprising rhodium and nickel supported on a carbon support and methyl iodide promoter with unsatisfactory results such as low methanol conversion, low selectivity and yield of acetic acid, increase of the by-product methane, and the like.

In the case of ammonia synthesis, for example, it has been known in the art that methanation of a mixed gas comprising carbon monoxide and hydrogen of 99% by volume or more may be performed at relatively low temperatures in the presence of a catalyst comprising nickel or the like.

However, methanation of a mixed gas comprising high content carbon monoxide and low content hydrogen by use of a conventional catalyst comprising nickel or the like causes problems due to decomposition of carbon monoxide resulting in deposition of carbon, reduction in yield of carbon monoxide, deterioration of the catalyst and in blocking in the reactor. Furthermore the use of nickel catalyst results in formation of nickel carbonyl which may sublime, and strong adsorption of carbon monoxide onto the surface of the catalyst results in hindering the reaction.

It is an object of the present invention to provide a process for preparing acetic acid from methanol and carbon monoxide or a gaseous mixture of carbon monoxide with hydrogen, which process is capable of preparing acetic acid at high selectivity and yield with markedly reduced corrosion in the reaction system and markedly reduced by-produced methane by-product, by a simplified preparation process.

The present invention provides a process for preparing acetic acid by contacting methanol and a carbon monoxide component in the presence of a carbon-supported rhodium catalyst characterized in that the process comprises contacting methanol with the carbon monoxide component which is (a) carbon monoxide or (b) a mixed gas of carbon monoxide with hydrogen of 2% by volume or less or (c) a mixed gas of carbon monoxide with water (the water being in an amount of 3 to 50 mol % relative to the methanol) in the presence of the carbon-supported rhodium metal catalyst and a methyl iodide promoter in the vapour phase, under the conditions of a reaction temperature of 180-220°C, a reaction pressure of 5-10 kg/cm².G, and a weight of catalyst to feed gas flow ratio (W/F) of 10-20 g.h/mole in cases (a) or (b) or 1-30 g.h/mole in case (c).

There may be a preliminary step of subjecting an initial gaseous mixture of carbon monoxide with a high content of hydrogen to a catalytic methanation reaction in the presence of a catalyst prepared by supporting a platinum group metal on a heat-resistant support to obtain the gaseous mixture of carbon monoxide with hydrogen, with a hydrogen content of 2% by volume or less.

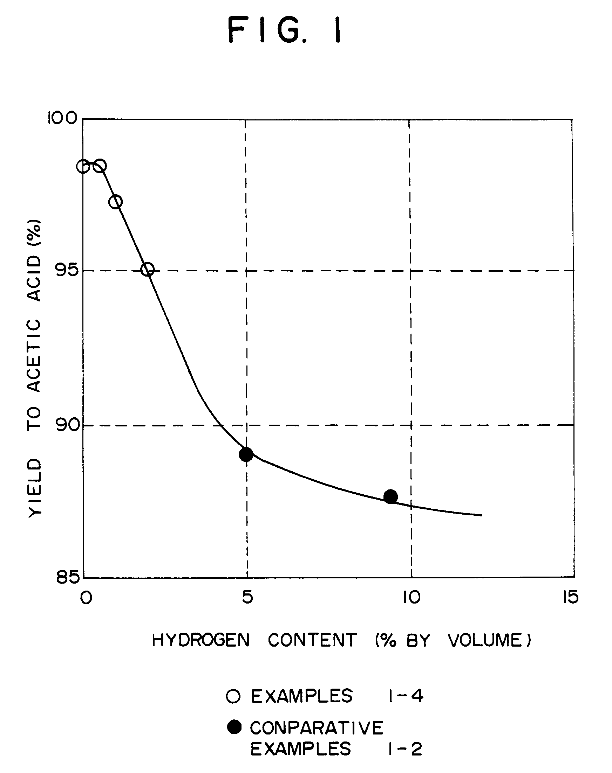

In the drawing Fig. 1 is a graph showing relationship between yields of acetic acid and hydrogen contents in Examples 1-4 and Comparative Examples 1-2.

Examples of the gaseous mixture of carbon monoxide with high content hydrogen used in a preferred embodiment of the present invention may include the so-called synthesis gas obtained by partial combustion, steam reforming, etc. of coal, petroleum and natural gas and containing 20 to 75% by volume of hydrogen, a mixed gas obtained by reducing carbon dioxide under hydrogen and containing 5 to 75% by volume of hydrogen, and the like.

Examples of the process for preparing the gaseous component containing 2% by volume or less, preferably 1% by volume of hydrogen may include known processes such as cuprammonium process, COSORB process, deep freeze separation process, adsorption process utilizing the selective carbon monoxide-adsorbing power of an adsorbent prepared by covering activated alumina with carbon followed by CuCl and CuCl$_2$, i.e. a pressure swing adsorption process or the like, and preferably may include the following methanation process and combinations of the methanation process with the above known processes.

The above methanation process comprises introducing the above mixed gas of carbon monoxide with high content hydrogen into a reactor filled with a catalyst prepared by supporting a metal belonging to platinum group, preferably platinum on a heat-resistant support, preferably γ-alumina for carrying out a catalytic methanation reaction to remove hydrogen in the mixed gas.

The above methanation process is essentially different from the conventional methanation process for removing small amount of carbon monoxide and carbon dioxide by use of a nickel catalyst or the like in that the former is directed to a mixed gas containing carbon monoxide as a major component, whereas the latter is directed to a mixed gas containing hydrogen as a major component.

The carbon in the carbon-supported rhodium metal catalyst of the present invention may itself be supported on an inorganic support such as silica, alumina, zeolite, and the like, or may be for example activated carbon, carbon black or coke. Of these, activated carbon is preferred.

The amount of rhodium metal to be supported in the carbon-supported rhodium metal catalyst is not specifically limited, but usually in the range of 0.01 to 20% by weight, preferably 0.1 to 10% by weight, more preferably 1 to 5% by weight based on the weight of the catalyst.

The carbon-supported rhodium metal catalyst used in the process of the present invention may be prepared by a process which comprises supporting a rhodium reagent on a carbon support according to impregnation process, deposition process, immersion process, metallizing process, kneading process, etc., followed by evaporating water for drying at 50 to 200°C, preferably 80 to 120°C, and by flowing a hydrogen-containing gas through the catalyst at 100 to 700°C, preferably 200 to 400°C for reduction under hydrogen atmosphere. Reduction for the preparation of the catalyst may also be carried out in the liquid phase by use of a combination of formalin with alkali.

Examples of the above rhodium reagent may include rhodium chloride, sodium chlororhodate, ammonium chlororhodate, rhodium hydroxide and the like. Of these, rhodium chloride is particularly preferred.

Since the reduction for the preparation of the catalyst may be carried out during the preparation of acetic acid too, the above reduction step is not always necessary.

The amount of methyl iodide used in the process of the present invention is not specifically limited, but is usually in the range of 0.1 to 50 moles, preferably 1 to 20 moles per 100 moles of methanol. Iodine compounds such as iodine, hydrogen iodide, which are capable of forming methyl iodide by reaction with methanol in the reaction system, may also be used as the promoter in place of methyl iodide.

The catalytic reaction in the second aspect of the present invention is carried out in the presence of water. The presence of water in the catalytic reaction according to the second aspect of the present invention increases selectivity to acetic acid and decreases selectivity to by-produced methane, resulting in greatly increasing a ratio between selectivity to acetic acid and selectivity to by-produced methane (hereinafter simply referred to as a selectivity ratio), increasing yield of acetic acid and in reducing unnecessary consumption of methanol.

The by-production of methane may be reduced, as the amount of water used in the catalytic reaction is increased. Preferably, the amount of water used in the catalytic reaction is in the range of from 3 to 50 mol% relative to methanol used. When the above amount is less than 3 mol%, the effect of reducing by-

3

production of methane is unsatisfactory. On the other hand, when the above amount is more than 50 mol%, partial pressures of carbon monoxide and methanol in the reaction system are so reduced that methanol conversion and yield to acetic acid are undesirably reduced.

The catalytic reaction in the first aspect of the present invention is carried out by contacting methanol with carbon monoxide or with a mixed gas of carbon monoxide and hydrogen of 2% by volume or less, preferably 1 % by volume or less in the presence of the above carbon-supported rhodium metal catalyst and methyl iodide promoter in vapor phase under conditions of a methanol to carbon monoxide molar ratio of 1:100 to 100:1, preferably 1:10 to 10:1, a reaction temperature of 100 to 400°C, preferably 150 to 300°C, more preferably 180 to 220°C, a reaction pressure of -0.9 to 300 $kg/cm^2 \cdot G$, preferably 0 to 100 $kg/cm^2 \cdot G$, more preferably 5 to 10 $kg/cm^2 \cdot G$, and a weight of catalyst to feed gas flow rate ratio (W/F) of 0.1 to 500 g•h/mole, preferably 1 to 30 g•h/mole, more preferably 10 to 20 g•h/mole.

The catalytic reaction in the second aspect of the present invention may be carried out by contacting methanol with carbon monoxide in the presence of a carbon-supported rhodium metal catalyst, a methyl iodide promoter and water in vapor phase preferably under the conditions of: a methanol to carbon monoxide molar ratio of 1:100 to 100:1, more preferably 1:10 to 10:1, a reaction temperature of 100 to 400°C, preferably 150 to 300°C, a reaction pressure of -0.9 to 300 $kg/cm^2.G$, preferably 0 to 100 $kg/cm^2.G$, and a weight of the catalyst to feed gas flow rate ratio (W/F) of 0.1 to 500 g.h/mole, preferably 1 to 30 g.h/mole.

The catalytic action in the above catalytic reaction is not hindered by the presence of methane as an impurity.

When the above reaction temperature is higher than 400°C, by-production of methane is undesirably increased.

The reactor used in the catalytic reaction according to the process of the present invention may be of fixed catalyst bed type, fluidized bed type or moving bed type.

In principle, the reaction of methanol with carbon monoxide is effected so as to form methyl acetate and water at a first step, followed by reaction of the methyl acetate with water to form acetic acid and methanol, which is recirculated to be used as a starting material. If hydrogen presents under the above reaction conditions as in the first aspect of the present invention, the hydrogen reacts with methyl acetate to form acetic acid and methane, resulting in reducing yield to acetic acid. On the other hand, if hydrogen presents under the above reaction conditions, a reaction of the methyl iodide promoter with hydrogen causes decomposition of methyl iodide to take place, resulting in hindering the reaction for the preparation of acetic acid and in causing corrosion in the apparatus for the preparation of acetic acid because of the corrosive properties of decomposition products of methyl iodide, i.g. iodine and hydrogen iodide.

The present invention makes it possible to provide a process for preparing acetic acid from methanol and carbon monoxide or a mixed gas of carbon monoxide with hydrogen, which is capable of providing such advantages that unnecessary consumption of methyl iodide promoter is reduced so as not to hinder the reaction for the formation of acetic acid, that by-production of methane is so reduced that a recycle gas comprising unreacted carbon monoxide for recovery contains such a small amount of methane as to be reused as a starting material without needing methane separation process, and that acetic acid is prepared at high selectivity and high yield.

The present invention will be explained more in detail by the following Examples and Comparative Examples.

Examples 1-4

A granular activated carbon (marketed by Takeda Chemical Industries, LTD. under a trade name of Shirasagi C2C) was screened and classified into a particle size range of 20 to 42 meshes, i.e. 0.35 to 0.84 mm in particle size to be weighed by 15.21 g. Next, rhodium chloride was weighed by such an amount that 2.5% by weight of rhodium metal may be supported on the above activated carbon support and dissolved in water to form an aqueous solution of 100 cc. The activated carbon weighed as above was immersed in the above aqueous solution, followed by evaporating water on a water bath by use of a rotary evaporator, drying in a dryer at 120°C, and reducing under hydrogen atmosphere at 400°C for 2 hours to obtain an activated carbon-supported rhodium metal catalyst to be used. A fixed catalyst bed flow type reaction apparatus comprising a device for feeding methanol and a gaseous component as a starting material, a methanol evaporator, a feed preheater, a reactor having an inner diameter of 10 mm, a cooler and an acetic acid-recovering device was used as a reaction apparatus.

A highly purified carbon monoxide having a purity of 99.95% (Example 1) and mixed gases of the highly purified carbon monoxide with hydrogen of 0.5% by volume (Example 2), 1% by volume (Example 3) and 2% by volume (Example 4) respectively were prepared.

Methanol and methyl iodide as starting materials were fed into the evaporator at a predetermined flow rate to be vaporized, and a gaseous component as a starting material at a predetermined flow rate was mixed with the vaporized methanol and methyl iodide to be heated up to 160°C in the feed preheater, followed by introducing into the reactor filled with 6 g of the activated carbon-supported rhodium metal catalyst for carrying out a catalytic reaction under the conditions shown in Table 1, and cooling the reactor effluents to recover acetic acid according to gas-liquid separation process. The results are shown in Table 2 and Fig. 1.

Comparative Examples 1-2

The procedures of Example 1 were repeated except for the conditions shown in Table 1. The results are shown in Table 1 and Fig. 1.

Comparative Examples 3-5

The procedures of Example 1 were repeated except for the conditions shown in Table 1. The results are shown in Table 2.

Examples 5-6

The procedures of Example 1 were repeated except for the conditions shown in Table 1. The results are shown in Table 2.

## Table 1

| | Reaction temperature (°C) | Reaction pressure (kg/cm²·G) | W/F (g·h/mol) | $H_2$ content (vol %) | $CO + H_2$:methanol:$CH_3I$ (molar ratio) |
|---|---|---|---|---|---|
| Example 1 | 200 | 9 | 15 | 0 | 100:20:1 |
| " 2 | 200 | 9 | 15 | 0.5 | 100:20:1 |
| " 3 | 200 | 9 | 15 | 1.0 | 100:20:1 |
| " 4 | 200 | 9 | 15 | 2.0 | 100:20:1 |
| " 5 | 200 | 5 | 15 | 0 | 100:20:1 |
| " 6 | 200 | 9 | 10 | 0 | 100:20:1 |
| Comparative Example 1 | 200 | 9 | 15 | 5.0 | 100:20:1 |
| " 2 | 200 | 9 | 15 | 9.4 | 100:20:1 |
| " 3 | 150 | 9 | 5 | 0 | 100:20:1 |
| " 4 | 250 | 9 | 5 | 0 | 100:20:1 |
| " 5 | 200 | 9 | 5 | 0 | 100:20:1 |

EP 0 526 974 B1

### Table 2

| | Methanol conversion (%) | Selectivity (%) | | Percentage of by-produced methane (%) | Yield to acetic acid (%) |
|---|---|---|---|---|---|
| | | Acetic acid | Methane | | |
| Example 1 | 99.99 | 98.69 | 0.23 | 0.23 | 98.5 |
| " 2 | 99.99 | 98.72 | 0.68 | 0.68 | 98.5 |
| " 3 | 99.99 | 97.69 | 1.50 | 1.50 | 97.3 |
| " 4 | 99.99 | 95.92 | 2.72 | 2.69 | 95.1 |
| " 5 | 99.96 | 90.50 | 0.13 | 0.13 | 90.27 |
| " 6 | 99.91 | 93.27 | 0.13 | 0.13 | 93.00 |
| Comparative Example 1 | 99.99 | 90.96 | 6.45 | 6.34 | 89.4 |
| " 2 | 99.99 | 88.41 | 10.66 | 10.56 | 87.6 |
| " 3 | 97.44 | 49.84 | 0.04 | 0.03 | 47.8 |
| " 4 | 99.99 | 95.49 | 2.19 | 2.18 | 95.08 |
| " 5 | 99.78 | 77.64 | 0.12 | 0.12 | 77.07 |

Comparative Example 6

The procedures of Example 1 were repeated except that an activated carbon-supported nickel catalyst prepared by supporting 2.5% by weight of nickel metal by use of nickel acetate in place of rhodium chloride on the activated carbon support was used in place of the activated carbon-supported rhodium metal catalyst with the results that yield to acetic acid was as low as 9.6% and percentage of by-produced methane was 0.63 % when carbon monoxide was used as the gaseous component, and that yield to acetic acid was improved to be 38.8% but lower compared with that of the activated carbon-supported rhodium metal catalyst, and percentage of by-produced methane was increased to be 3.33% when a mixed gas of carbon monoxide with hydrogen of 9.4% by volume was used as the gaseous component.

Example 7

The procedures of Example 1 were repeated except for the conditions shown in Table 3. The results are shown in Table 4.

Comparative Example 7

The procedures of Example 7 were repeated except for the conditions shown in Table 3. The results are shown in Table 4.

Table 3

| | Reaction temperature (°C) | Reaction pressure (kg/cm²·G) | W/F (g·h/mol) | CO:methanol:CH$_3$I (molar ratio) | Water/methanol (molar ratio) |
|---|---|---|---|---|---|
| Example 7 | 200 | 9 | 5 | 100:20:1 | 0.0965 |
| Comparative Example 7 | 200 | 9 | 5 | 100:20:1 | 0 |

Table 4

| | Methanol conversion (%) | Selectivity (%) Acetic acid (b) | Selectivity (%) Methane (a) | Selectivity ratio (b/a) | Percentage of by-produced methane (%) | Yield to acetic acid (%) |
|---|---|---|---|---|---|---|
| Example 7 | 99.72 | 77.81 | 0.08 | 973 | 0.08 | 77.2 |
| Comparative Example 7 | 99.78 | 77.64 | 0.12 | 647 | 0.12 | 77.1 |

## Claims

1. A process for preparing acetic acid by contacting methanol and a carbon monoxide component in the presence of a carbon-supported rhodium catalyst characterized in that the process comprises contacting methanol with the carbon monoxide component which is (a) carbon monoxide or (b) a mixed gas of carbon monoxide with hydrogen of 2% by volume or less or (c) a mixed gas of carbon monoxide with water (the water being in an amount of 3 to 50 mol % relative to the methanol) in the presence of the carbon-supported rhodium metal catalyst and a methyl iodide promoter in the vapour phase, under the conditions of a reaction temperature of 180-220 °C, a reaction pressure of 5-10 $kg/cm^2$.G, and a weight of catalyst to feed gas flow ratio (W/F) of 10-20 g.h/mole in cases (a) or (b) or 1-30 g.h/mole in case (c).

2. A process according to claim 1 wherein the gaseous mixture (b) is used and has a hydrogen content of 1% by volume or less.

3. A process according to claim 1 or claim 2 wherein the gaseous mixture of carbon monoxide with hydrogen is used and which process comprises the preliminary step of subjecting an initial gaseous mixture of carbon monoxide with a high content of hydrogen to a catalytic methanation reaction in the presence of a catalyst prepared by supporting a platinum group metal on a heat-resistant support to obtain the gaseous mixture of carbon monoxide with hydrogen, with a hydrogen content of 2% by volume or less.

4. A process as claimed in claim 3 wherein the initial gaseous mixture contains 5 to 75% by volume of hydrogen.

5. A process according to claim 3 or claim 4 wherein the initial gaseous mixture is a synthesis gas.

## Patentansprüche

1. Verfahren zur Herstellung von Essigsäure durch In-Kontakt-Bringen von Methanol und einer Kohlenmonoxid-Komponente in Gegenwart eines Rhodiumkatalysators auf einem Kohlenstoff-Träger, dadurch gekennzeichnet, daß das Verfahren das In-Kontakt-Bringen von Methanol mit der Kohlenmonoxid-Komponente, die (a) Kohlenmonoxid oder (b) ein Mischgas aus Kohlenmonoxid und 2 Vol.-% oder weniger Wasserstoff oder (c) ein Mischgas aus Kohlenmonoxid und Wasser ist (wobei das Wasser in einer Menge von 3 - 50 Mol-%, bezogen auf Methanol, vorliegt), in Gegenwart des Katalysators aus metallischem Rhodium auf einem Kohlenstoff-Träger und eines Methyliodid-Promotors in der Dampfphase unter den Bedingungen einer Reaktionstemperatur von 180 - 220 °C, eines Reaktionsdrucks von 5 - 10 $kg/cm^2$ und eines Verhältnisses von Katalysatorgewicht zum Feed-Gasstrom (W/F) von 10 - 20 g.h/Mol in den Fällen (a) und (b) oder 1 - 30 g.h/Mol im Fall (c), umfaßt.

2. Verfahren nach Anspruch 1, worin das Gasgemisch (b) eingesetzt wird und einen Wasserstoffgehalt von 1 Vol.-% oder weniger aufweist.

3. Verfahren nach Anspruch 1 oder 2, worin das Gasgemisch aus Kohlenmonoxid und Wasserstoff eingesetzt wird, umfassend den ersten Schritt des Unterwerfens eines Ausgangsgasgemischs aus Kohlenmonoxid mit einem hohen Wasserstoffgehalt einer katalytischen Methanbildungsreaktion in Gegenwart eines Katalysators, der durch Aufbringen eines Platingruppenmetalls auf einen hitzebeständigen Träger hergestellt wird, um das Gasgemisch aus Kohlenmonoxid und Wasserstoff mit einem Wasserstoffgehalt von 2 Vol.-% oder weniger zu erhalten.

4. Verfahren nach Anspruch 3, worin das Ausgangsgasgemisch 5 - 75 Vol.-% Wasserstoff enthält.

5. Verfahren nach Anspruch 3 oder 4, worin das Ausgangsgasgemisch ein Synthesegas ist.

## Revendications

1. Procédé de préparation d'acide acétique par mise en contact du méthanol et d'un composant d'oxyde de carbone, en présence d'un catalyseur de rhodium supporté sur du carbone, caractérisé en ce que le procédé comprend la mise en contact du méthanol avec le composant d'oxyde de carbone, qui est (a) l'oxyde de carbone ou (b) un gaz mixte d'oxyde de carbone avec de l'hydrogène à 2% en volume ou moins, ou (c un gaz mixte d'oxyde de carbone avec de l'eau (l'eau étant en une quantité de 3 à 50% en moles relativement au méthanol), en présence du catalyseur de rhodium métallique supporté sur du carbone et d'un promoteur d'iodure de méthyle dans la phase vapeur, dans les conditions d'une température de réaction de 180-220°C, d'une pression de réaction de 5-10 kg/cm$^2$.G, et d'un rapport du poids et du catalyseur au débit du gaz d'alimentation (P/D) de 10-20 g.h/mole dans les cas (a) ou (b) ou de 1-30 g.h/mole dans le cas (c).

2. Procédé selon la revendication 1, où le mélange gazeux (b) est utilisé et a une teneur en hydrogène de 1% en volume ou moins.

3. Procédé selon la revendication 1 ou la revendication 2, où le mélange gazeux d'oxyde de carbone avec de l'hydrogène est utilisé et ce procédé comprend l'étape préliminaire de soumettre un mélange gazeux initial d'oxyde de carbone avec une forte teneur en hydrogène à une réaction de méthanation catalytique, en présence d'un catalyseur préparé en supportant un métal du groupe platine sur un support thermo-résistant, pour obtenir le mélange gazeux d'oxyde de carbone avec de l'hydrogène, avec une teneur en hydrogène de 2% en volume ou moins.

4. Procédé selon la revendication 3, où le mélange gazeux initial contient de 5 à 7% en volume d'hydrogène.

5. Procédé selon la revendication 3 ou la revendication 4, où le mélange gazeux initial est un gaz de synthèse.

# FIG. I

○ EXAMPLES  I-4

● CONPARATIVE
  EXAMPLES  I-2